Europäisches Patentamt

(19) European Patent Office

Office européen des brevets

(11) Publication number: **0 081 975**
**B1**

## (12) EUROPEAN PATENT SPECIFICATION

(45) Date of publication of patent specification: **26.11.86**

(21) Application number: **82306554.5**

(22) Date of filing: **08.12.82**

(51) Int. Cl.⁴: **A 61 B 10/00,** A 61 B 17/20, A 61 M 37/00

(54) Device and method for allergy testing.

(30) Priority: **14.12.81 US 330587**
**30.11.82 US 445746**

(43) Date of publication of application:
**22.06.83 Bulletin 83/25**

(45) Publication of the grant of the patent:
**26.11.86 Bulletin 86/48**

(84) Designated Contracting States:
**AT BE CH DE FR GB IT LI LU NL SE**

(56) References cited:
**GB-A-1 459 262**
**US-A-2 893 392**
**US-A-3 136 314**
**US-A-3 814 097**
**US-A-3 964 482**
**US-E- 25 637**

(73) Proprietor: **Maganias, Nicholas H.**
**Reston Medical Building 1712 Club House Road**
**Reston Virginia 22090 (US)**

(72) Inventor: **Maganias, Nicholas H.**
**Reston Medical Building 1712 Club House Road**
**Reston Virginia 22090 (US)**

(74) Representative: **Burford, Anthony Frederick et al**
**W.H. Beck, Greener & Co. 7 Stone Buildings Lincoln's Inn**
**London WC2A 3SZ (GB)**

Courier Press, Leamington Spa, England.

## Description

This invention relates to a device for allergy testing the skin of a patient.

Testing the skin of a patient for an allergic reaction to any of a variety of allergens is a known technique involving penetration of the allergen into a small area of the skin and subsequently visually observing the reaction, if any, of the skin to the allergen. Known procedures for applying the allergen include the scratch test, the prick test and the intradermal test. The scratch test is carried out by applying a few drops of the allergen to the skin and slightly abrading the skin by scratching at that location. The prick test is similar except that abrasion of the skin is effected by making a plurality of pricks with a sharp needle. The intradermal test is carried out by injecting the allergen into the skin.

United States patents relating to the introduction of biochemical substances into the skin include Krug et al. 3,289,670; Kravitz et al. Re. 25,637, 2,817,336, 3,062,212, 3,136,314 and 3,351,059; Wager et al. 2,893,392, Ganderton et al. 3,814,097 and Gerstel et al. 3,964,482.

The present invention is based in part on the observation that each of the scratch test, the prick test and the intradermal test has one or more disadvantages. The scratch test may not produce enough scarification of the skin and thus lead to an inaccurate conclusion due to insufficient skin cell damage to permit adequate entry of and reaction with the allergen. The prick test may produce excessive trauma, for example, microbleeding, which can produce histamine release thereby confusing the results of the test. With the intradermal test, it is frequently difficult to titrate the degree of skin reaction and large and often false positive reactions develop. Each procedure has its own advantages, however. The medical practitioner will generally decide on which procedure to use taking into consideration the time required for the test, pain inflicted on the patient, trauma to the skin, weak reactions, expense and other features.

According to one aspect of the present invention, there is provided a test strip for allergy testing by a technique involving penetration of an allergen into the skin, said test strip comprising a flexible strip of tape having one of its surfaces coated with an adhesive and at least one skin-penetrating lance projecting from said one surface, characterised in that said at least one lance projects from a substrate having a concave cavity for containing the allergen.

Another aspect of the present invention provides a lance device for use in a test strip of the invention comprising at least one penetrating lance projecting from a surface of the device, characterised in that said surface is recessed defining an open-ended cavity for retaining the allergen and said at least one lance projects through the open end of the cavity.

US—A—3136314 and US—E—25637 disclose devices for intracutaneous injections, especially vaccination, in which one or more skin-penetrating lances project from a rigid substrate. The device of US—A—3136314 has a planar substrate and is provided within a sealed envelope containing a biological liquid and is used to penetrate through the envelope and the epidermis when the envelope is pressed against the skin of a patient. The device of US—E—25637 is carried on an adhesive strip for securing the device on the skin of a patient. In one embodiment, the device has a planar substrate and is applied to an area of the skin of a patient to which a vaccine has been applied to scarify said area. In another embodiment, the substrate is domed with the lances projecting from the convex surface thereof and is perforate. A frangible or puncturable capsule containing a liquid vaccine is provided adjacent the concave surface of the substrate so that, on pressing the device against the skin, the capsule is broken or ruptured to release the vaccine, which then flows through substrate onto the skin. Needle-like projections can be provided on the concave surface of the substrate to pierce the capsule.

Neither US—A—3136314 nor US—E—25637 disclose directly loading the substrate or lances with liquid nor do they disclose the use of the devices with allergens.

The allergy testing device of the invention is used in a method, which combines a number of the advantages of the usual procedures while avoiding or reducing the disadvantages referred to above. The method involves applying to the skin of the patient a test strip in the form of a thin flexible piece of adhesive tape, a portion of which carries on its adhesive side one or more projecting lances coated with or supplied with the allergen and capable of piercing the skin to a limited degree. The test strip, and in particular the portion carrying the lance or lances, is then pressed against the skin to cause the lance or lances to penetrate the skin to a depth less than the subcutaneous tissue thereby carrying some of the allergen into the skin and maintaining constant contact between the allergen and the traumatized skin cells. After a period of time appropriate for the skin to react with the allergen, for example 15 to 20 minutes, the test strip including the lance or lances is removed from the skin and discarded. The degree of skin reaction, in terms of inflammation, swelling or the formation of protuberances, and hence the degree of allergy is then determined by the medical practitioner by visually observing the area of skin which was punctured by the lance or lances. Several such tapes, each carrying a different allergen, can be applied to the skin simultaneously or essentially simultaneously. Alternatively a single tape can carry a plurality of lances or groups of lances, each lance or group carrying a different allergen. The appearance of the skin area may be compared to one or more control skin areas which have been similarly punctured by similar test strips having a lance or lances free of allergen.

The lance or lances, which may be made of plastics material or metal, may be needle-like with sharp points, conical with sharp points or blade-like with both edges sharpened and a pointed end or rounded knife-edge end. In one embodiment there is at least one group of closely-spaced short needle-like lances, the group being for example circular and having a diameter less than the smallest dimension of the adhesive surface of the strip. A suitable arrangement is 5 to 10 lances disposed in a circular group of approximately 0.125 inch (3.175 mm) diameter. The length of the lances may be in the range 0.06 inch to 0.03 inch (1.5 mm to 0.75 mm).

In another embodiment, the test strip may carry a single lance or several rather widely spaced-apart lances. In this case, the single lance should be more massive than the smaller lances used in the plural-lance embodiment in order to provide the necessary skin damage. A suitable shape for such a lance is conical with a sharp point. An advantage of the single lance construction is that the single lance will provide better penetration as well as more damage to the skin cells with resulting better allergic reaction. A single lance can also provide better attachment to the skin for longer application periods.

The preferred shape of the strip is rectangular, somewhat smaller than the conventional adhesive bandage applied to minor cuts and scratches, although square, circular, oblong or other shapes are satisfactory. It has been found that test strips of this kind when used as described above do not produce excessive trauma or microbleeding, while at the same time they produce reliable test results due in part to the fact that they maintain constant contact between the allergen and the traumatized skin cells during the test.

The test strip and the adhesive material are made of low allergenic material. Also, the strip should be colorless and transparent so that the reaction of the skin can be observed through the strip and so that, in the event of over-reaction, the test can be interrupted by removing the strip from the skin. The adhesive area of the strip will always be sufficiently great to adhere well to the skin.

The allergen initially applied to the lances may be in liquid form or solid form. If the allergen is dry, distilled water may be added shortly before use to dissolve or disperse the allergen and allow impregnation. The lance or lances may be covered with a removable protective cover to exclude air and dust and to maintain the lances in sterile condition until ready for use. The cover may be a plastic film overlying the lance or lances only or overlying the lances and the layer of adhesive. The allergen may of course be any allergen, such as those typically used for testing allergy to trees, grasses, weeds, cat hair, dog epithelium, house dust, molds, spores and certain inhalents.

The lance or lances may be held in their relative positions by having their inner ends secured to a substrate which in turn is attached to the adhesive tape. The substrate may be flexible or rigid and may be attached to the adhesive tape by means of the same layer of adhesive which adheres the tape to the patient's skin. The substrate can be concave so as to provide an outwardly facing cavity for retaining the liquid allergen. In the preferred construction the cavity is relatively deep and of predetermined capacity to hold the appropriate volume of allergen when filled to the rim. The cavity thus serves to standardize the volume of allergen applied to the skin.

For the convenience of the medical practitioner the allergy testing strips can be supplied in kit form, with each strip being marked to identify its respective allergen or allergens. The kit may also include control strips having no allergen applied to their lances. Use of the strips by the practitioner is convenient, as no instruments are required and as the strips are discarded after use.

Brief Description of the Drawings

Figure 1 is a bottom view of the test strip embodying the principles of the present invention;

Figure 2 is a sectional view taken on the line 2—2 of Figure 1; and

Figure 3 is a sectional view of a single-lance type of test strip.

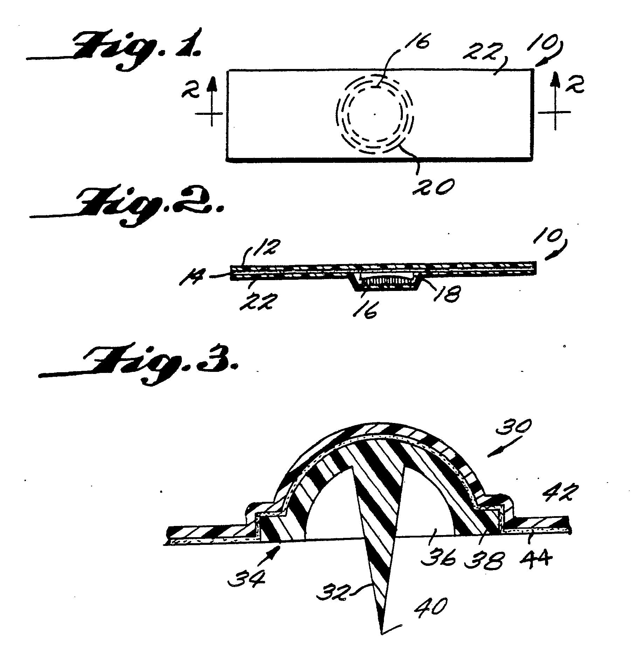

The thickness of the layers in Figures 2 and 3 is exaggerated for clarity of illustration.

Figures 1 and 2 illustrate an allergy test strip 10 which includes a strip of flexible adhesive tape in the form of a plastic strip 12 having on one surface a layer of pressure-sensitive adhesive 14. Preferably the strip 12 is clear, i.e., essentially colorless, and is sufficiently transparent that a skin reaction can be observed through it. The adhesive 14 is also essentially transparent and colorless. Projecting from the adhesive surface is a group of pointed or bladed lances 16 which can carry an allergen (not illustrated) on their surfaces and/or in the interstices between them. The inner ends of the lances 16 are attached to a substrate 18 which in turn is attached to the film 12 by means of the adhesive layer 14. The substrate 18 is relatively rigid compared to the adhesive tape and is concave outwardly to form a cavity or well for retaining liquid allergen. A removable protective cover (not shown) can overlay the group of lances 16 and be sealed in place at its periphery by contact with the adhesive layer 14. The cover could alternatively be relatively rigid or elastic and held in place by frictional engagement with the periphery of the group of lances. A removable protective film 22 overlies the lances and, if present, the said cover and the exposed adhesive and is held in place by the latter. In the illustrated embodiment the test strip 10 is rectangular and the group of lances is circular, these being the preferred shapes.

To use the test strip the medical practitioner first removes the protective film and, if present, the underlaying cover. If the allergen is in liquid form no further preparation is required. If the allergen is in dry form the practitioner will apply several drops of sterile water to the group of lances 16 to dissolve or disperse the allergen and allow it to impregnate the spaces between the lances. After sterilizing the skin portion selected for the allergy test, the practitioner applies the adhesive side of the test strip 10 to the skin to hold the strip in place. Simultaneously or subsequently he gently and firmly presses with his fingers against the strip opposite the group of lances to cause the latter to penetrate the skin. The liquid allergen is thus carried partly into the skin and is maintained in constant contact with the traumatized skin cells until the strip is removed. A contact time of 15—20 minutes is generally suitable and upon removal of the strip the practitioner observes the punctured skin area to determine the extent of allergic reaction. The punctured skin area can be compared to an adjacent control area which has been punctured by the allergen-free lances of a control test strip.

Figure 3 illustrates a test strip 30 which includes only a single lance 32 or a plurality of such lances spaced apart sufficiently that a skin reaction caused by one lance is independent of a skin reaction caused by another lance. In the illustrated construction the lance 32 is molded from plastics material integrally with a substrate 34 which forms a concave recess 36 around the base of the lance 32. The substrate 34 is circular in plan view and is dome-shaped with a peripheral flange 38 which surrounds the generally semi-spherical recess 36. The lance 32 projects through the center of the recess 36 and is conical in shape with a sharp point 40. Suitable dimensions for the substrate 34 are an overall diameter of about 0.3 inches (7.5 mm), an overall height of about 0.125 inches (3.175 mm) and a flange and wall thickness of about 0.03 inches (0.75 mm). The conical lance 32 may have a taper of 16°.

As with the embodiment of Figure 2, the test strip 30 of Figure 3 includes a plastic strip 42, preferably essentially colorless and transparent, coated on its lower surface with a layer of pressure-sensitive adhesive 44. While not shown in Figure 3, the test strip 30 can be provided with a protective film (as shown at 22 in Figure 2), and the lance 32 can be provided with a cover.

The concave substrate of Figure 2 and the recess 36 in Figure 3 serve to standardize the amount of allergen applied to the skin.

**Claims**

1. A test strip for allergy testing by a technique involving penetration of an allergen into the skin, said test strip comprising a flexible strip of tape (12, 42) having one of its surfaces coated with an adhesive (14, 44) and at least one skin-penetrating lance (16, 32) projecting from said one surface, characterised in that said at least one lance projects from a substrate having a concave cavity (18, 36) for containing the allergen.

2. A test strip as claimed in Claim 1, wherein the cavity contains an allergen.

3. A test strip as claimed in Claim 2, wherein the allergen is a dry allergen.

4. A test strip as claimed in any one of the preceding claims, wherein the cavity is provided in a rigid substrate (34) having a skin-contacting peripheral flange (38) surrounding the open end of said cavity.

5. A test strip as claimed in Claim 4, wherein the substrate has a generally semi-spherical dome forming the said cavity and said flange is generally circular.

6. A test strip as claimed in any one of the preceding claims, wherein said at least one lance is a single lance (32) of pointed conical shape.

7. A test strip as claimed in any one of Claims 1 to 5, wherein there are a plurality of closely-spaced, allergen-carrying lances (16) arranged in a group.

8. A test strip as claimed in any one of the preceding claims, wherein the lance or lances project from the cavity by a distance less than the thickness of subcutaneous tissue.

9. A test strip as claimed in Claim 8, wherein the lance or lances protrude from the cavity by 0.75 to 1.5 mm (0.03 to 0.06 inches).

10. A test strip as claimed in any one of the preceding claims, wherein it is essentially transparent whereby a visible allergic skin reaction can be observed through the test strip.

11. A lance device for use in a test strip as claimed in Claim 1 comprising at least one penetrating lance (16, 32) projecting from a surface of the device, characterised in that said surface is recessed defining an open-ended cavity (18, 36) for retaining the allergen and said at least one lance projects through the open end of the cavity.

12. A lance device as claimed in Claim 11, wherein the cavity is provided in a rigid substrate (34) having a skin-contacting peripheral flange (38) surrounding the open end of the said cavity.

13. A lance as claimed in Claim 12, wherein said substrate has a generally semi-spherical dome forming the said cavity and said flange is generally circular.

14. A lance device as claimed in any one of Claims 11 to 13, wherein said at least one lance is a single lance (32) of pointed conical shape.

15. A lance device as claimed in any one of Claims 11 to 13, wherein the lance or lances project from the cavity by a distance less than the thickness of subcutaneous tissue.

16. A lance device as claimed in Claim 15, wherein it is essentially transparent whereby a visible allergic skin reaction can be observed through the device.

**Patentansprüche**

1. Teststreifen zum Durchführen eines Allergietests bei dem ein Allergen in die Haut eindringt, wobei der Teststreifen einen flexiblen Klebstreifen (12, 42) aufweist, dessen eine Seite mit einem Klebstoff (14, 44) beschichtet ist und bei dem mindestens eine die Haut durchdringende Spitze (16, 32) von der einen Oberfläche vorsteht, dadurch gekennzeichnet, daß die mindestens eine Spitze von einem Träger vorsteht, der einen konkaven Hohlraum (18, 36) zur Aufnahme des Allergens hat.

2. Teststreifen nach Anspruch 1, dadurch gekennzeichnet, daß der Hohlraum ein Allergen enthält.

3. Teststreifen nach Anspruch 2, dadurch gekennzeichnet, daß das Allergen ein trockenes Allergen ist.

4. Teststreifen nach einem der vorhergehenden Ansprüche, dadurch gekennzeichnet, daß der Hohlraum in einem starren Träger (34) vorgesehen ist, der einen die Haut berührenden Umfangsflansch (38) besitzt, welcher das offene Ende des Hohlraums umgibt.

5. Teststreifen nach Anspruch 4, dadurch gekennzeichnet, daß das Substrat einen im allgemeinen halbkugelförmigen Dom aufweist, der den Hohlraum bildet und daß der Flansch im allgemeinen kreisförmig ist.

6. Teststreifen nach einem der vorhergehenden Ansprüche, dadurch gekennzeichnet, daß die mindestens eine Spitze eine einzige Spitze (32) von Spitzkegelform ist.

7. Teststreifen nach einem der Ansprüche 1 bis 5, dadurch gekennzeichnet, daß eine Anzahl von eng nebeneinander angeordneten, allergentragenden Spitzen (16) in einer Gruppe angeordnet ist.

8. Teststreifen nach einem der vorhergehenden Ansprüche, dadurch gekennzeichnet, daß die Spitze oder Spitzen von dem Hohlraum um eine Distanz vorstehen, die geringer als die Dicke des subkutanen Gewebes ist.

9. Teststreifen nach Anspruch 8, dadurch gekennzeichnet, daß die Spitze oder Spitzen aus dem Hohlraum 0,75 bis 1,5 mm vorstehen.

10. Teststreifen nach einem der vorhergehenden Ansprüche, dadurch gekennzeichnet, daß er im wesentlichen transparent ist, so daß eine sichtbare allergische Hautreaktion durch den Teststreifen beobachtet werden kann.

11. Spitzeneinrichtung zur Verwendung bei einem Teststreifen nach Anspruch 1, mit mindestens einer vorstehenden Spitze (16, 32), die von einer Oberfläche der Einrichtung vorsteht, dadurch gekennzeichnet, daß die Oberfläche ausgenommen ist und einen offenendigen Hohlraum (18, 36) bildet, um das Allergen aufzunehmen, und daß mindestens eine Spitze durch das offene Ende des Hohlraums vorsteht.

12. Spitzeneinrichtung nach Anspruch 11, dadurch gekennzeichnet, daß der Hohlraum in einem starren Träger (34) vorgesehen ist, der einen die Haut berührenden Umfangsflansch (38) aufweist, welcher das offene Ende des Hohlraums umgibt.

13. Spitzeneinrichtung nach Anspruch 12, dadurch gekennzeichnet, daß das Substrat einen im allgemeinen halbkugelförmigen Dom besitzt, der den Hohlraum bildet, und daß der Flansch im allgemeinen kreisförmig ist.

14. Spitzeneinrichtung nach einem der Ansprüche 11 bis 13, dadurch gekennzeichnet, daß die mindestens eine Spitze eine einzige Spitze (32) ist, die die Form eines spitzen Kegels hat.

15. Spitzeneinrichtung nach einem der Ansprüche 11 bis 13, dadurch gekennzeichnet, daß die Spitze oder die Spitzen von dem Hohlraum um eine Distanz vorstehen, die kleiner als die Dicke des subkutanen Gewebes ist.

16. Spitzeneinrichtung nach Anspruch 15, dadurch gekennzeichnet, daß sie im wesentlichen transparent ist, so daß eine sichtbare allergische Hautreaktion durch die Einrichtung beobachtet werden kann.

**Revendications**

1. Bande d'essai pour test allergique utilisant une technique comportant la pénétration d'un allergène dans la peau, ladite bande d'essai comprenant une bande souple de ruban (12, 42) dont une des surfaces est enduite d'un adhésif (14, 44) et où au moins une pointe de pénétration dans la peau (10, 32) fait saillie de ladite surface, caractérisée en ce que ladite au moins une pointe fait saillie d'un substrat comportant une cavité concave (18, 36) destinée à contenir l'allergène.

2. Bande d'essai selon la revendication 1, dans laquelle la cavité contient un allergène.

3. Bande d'essai selon la revendication 2, dans laquelle l'allergène est un allergène sec.

4. Bande d'essai selon l'une quelconque des revendications précédentes, dans laquelle la cavité est pratiquée dans un substrat (34) rigide ayant un rebord périphérique (38) de contact avec la peau entourant l'extrémité ouverte de ladite cavité.

5. Bande d'essai selon la revendication 4, dans laquelle le substrat a un dôme de forme générale semi-sphérique formant ladite cavité et ledit rebord est de forme générale circulaire.

6. Bande d'essai selon l'une quelconque des revendications précédentes, dans laquelle ladite au moins une pointe est une pointe (32) unique de forme conique pointue.

7. Bande d'essai selon l'une quelconque des revendications 1 à 5, dans laquelle plusieurs pointes (6) porteuses d'allergène peu espacées sont disposées en un groupe.

8. Bande d'essai selon l'une quelconque des revendications précédentes dans laquelle le ou les pointes font saillie de la cavité sur une distance inférieure à l'épaisseur du tissu sous-cutané.

9. Bande d'essai selon la revendication 8, dans laquelle la ou les pointes font saillie de la cavité sur 0,75 à 1,5 mm (0,03 à 0,06 pouce).

10. Bande d'essai selon l'une quelconque des revendications précédentes, qui est essentiellement transparente, grâce à quoi une réaction allergique visible de la peau peut être observée à travers la bande d'essai.

11. Dispositif à pointes destiné à être utilisé dans une bande d'essai selon la revendication 1 comportant au moins une pointe de pénétration (16, 32) faisant saillie depuis une surface du dispositif, caractérisé en ce que ladite surface est évidée et délimite une cavité à extrémité ouverte (18, 36) destinée à retenir l'allergène et ladite au moins une pointe fait saillie à travers la cavité.

12. Dispositif à pointes selon la revendication 11, dans lequel la cavité est prévue dans un substrat rigide (34) ayant un rebord périphérique (38) de contact avec la peau entourant l'extrémité ouverte de ladite cavité.

13. Dispositif à pointes selon la revendication 12, dans lequel ledit substrat a un dôme de forme générale semi-sphérique et ledit rebord est de forme générale circulaire.

14. Dispositif à pointes selon l'une quelconque des revendications 11 à 13, dans lequel ladite au moins une pointe est une pointe (32) unique de forme conique pointue.

15. Dispositif à pointes selon l'une quelconque des revendications 11 à 13, dans lequel la ou les pointes font saillie de la cavité sur une distance inférieure à l'épaisseur du tissu sous-cutané.

16. Dispositif à pointes selon la revendication 15, qui est essentiellement transparent, grâce à quoi une réaction allergique visible de la peau peut être observée à travers le dispositif.

*Fig. 1.*

*Fig. 2.*

*Fig. 3.*